# EUROPEAN PATENT APPLICATION

(11) **EP 2 202 244 A1**
(43) Date of publication of application: **30.06.2010**
(21) Application number: 08834126.8
(22) Date of filing: 01.09.2008
(51) Int. Cl.: C07K 16/18, G01N 33/53

(54) **METHOD OF IMMUNOLOGICAL ANALYSIS FOR DETECTION OF ANTIBODIES AGAINST HUMAN GSTT1 (ANTI-HGSTT1)**

(30) Priority: 27.09.2007 ES 200702641
(71) Applicant: Fundacion Reina Mercedes Para La Investigacion Sanitaria, 41013 Sevilla (ES)
(72) Inventor: NUÑEZ ROLDAN, Antonio, E-41013 Sevilla (ES); AGUILERA GARCIA, Isabel, E-41013 Sevilla (ES); WICHMANN SCHLIPF, Ingeborg, E-41013 Sevilla (ES)
(74) Representative: Temino Ceniceros, Ignacio
(86) International application number: PCT/ES2008/000572
(87) International publication number: WO 2009/040451

(57) **Abstract**

The subject of the present invention is a method of immunological analysis for detection in biological fluids of anti-bodies against human GSTT1 (anti-hGSTT1). The present invention also concerns the use of said immunological analysis method for the diagnosis, prognosis, follow-up and monitoring of pathological conditions associated with the presence of anti-GSTT1 in biological fluids, and also concerns a tool kit for putting said method into practice.

## Description

### TECHNICAL FIELD

The present invention is part of the medical and health technique, and its scope of application is that of analysis systems for diagnosing autoimmune processes and for preventing transplant rejection. It could likewise be of interest to researchers in the field of biomedical and clinical research.

### OBJECT OF THE INVENTION

The subject of the present invention is a method of immunological analysis for detection in biological fluids of antibodies directed against human GSTT1 (anti-hGSTT1). Likewise an object of the present invention is the use of said method of immunological analysis for the diagnosis, prognosis of onset, follow-up and monitoring of pathological conditions associated with the presence in biological fluids of anti-GSTT1, as well as a tool kit for implementing said method.

### STATE OF THE ART

Within the phenomenon of autoimmunity, research on the tolerance or rejection of cells or tissue in transplant patients has revealed the multifactorial nature of both processes. Thus, for example, the phenomena of allorecognition and alloimmune reaction have been identified, whereby genes expressed in the transplanted material would give rise to the appearance of anti-bodies (alloantibodies) in the recipient which would cause the rejection of certain transplants (Aguilera, I., et al (2001). Clin Exp Immunol, 126, 535-539). Recently, the existence of a new form of rejection of liver transplants has been described for the first time, which mainly affects children. This gives rise to an illness characterised by the presence of histological alterations typical of chronic hepatitis, a high concentration of IgG, the appearance of non- organic-specific antibodies and a response to immunosuppressive treatments. Hence its denomination as post transplant *de novo* hepatitis (Kerkar, N. et al (1998). Lancet, 351, 409-413; Jones, D.E., et al (1999). Hepatology, 30, 53-57). Due to the fact that clinically it is indistinguishable from other types of hepatitis, the differential diagnosis of post transplant *de novo* hepatitis is difficult and often requires a biopsy, with the consequent discomfort for the patient. The biopsies reveal infrequent histological alterations, such as bridge necrosis (porto-portal and porto-central), centrilobular damage and periportal interruptions, and, together with other serological, radiological and molecular biological data, help to confirm the diagnosis (Salcedo, M, et al (2002). Hepatology, 35, 349-356). Although the mechanisms that control its evolution are unknown, different studies support the hypothesis that post transplant *de novo* hepatitis is caused by an alloimmune-type response to an antigen expressed in the donor's organ (Aguilera, L., et al (2001). Clin Exp Immunol, 126, 535-539; Aguilera, I., et al (2004). Liver Transpl, 10, 1166-1172; Rodriguez-Mahou, M., et al (2007). Transplantation, 83, 1126-1129). Thus, a protein, glutathione-S-Transferasa T1 (GSTT1) has been identified, which appears to play an important part in the development of this new pathology, and acts as an allogen. The GSTT1 is an enzyme involved in cellular detoxification, with high levels of expression in erythrocytes, liver, kidney and other tissue. This protein is codified by a simple polymorphic gene which has two alleles, positive or null, which explains its absence in 20% of the Caucasian population and between 11 and 58% in individuals from other ethnic origins (Sprenger, R., et al (2000). Pharmacogenetics, 10, 557-565; Landi, S., (2000). Mutat Res, 463, 247-283). Studies in patients receiving a transplanted liver with clinical symptoms and analytical signs typical of post transplant *de novo* hepatitis reveal the presence of high levels of antibodies directed against GSTT1 (anti-GSTT1). Genetic analysis of these cases has shown that the donor was GSTT1 positive and, consequently, the transplanted liver expressed this enzyme. On the contrary, the receptor was lacking the GSTT1 gene (Aguilera, I., et al (2004). Liver Transpl., 10, 1166-1172; Rodriguez-Mahou, M., et al (2007). Transplantation, 83, 1126-1129). This research strengthens the alloimmune hypothesis of post transplant *de novo* hepatitis; it presents GSTT1 incompatibility as a fundamental element in its development, and indicates anti-GSTT1 antibodies as transplant rejection markers. Moreover, the presence of anti-GSTT1 antibodies has been demonstrated in patients who have not received any solid organ, but who, at one time, required a blood transfusion or were pregnant (Aguilera, I., et al (2005). Transplantation Proceedings, 37, 1457-1458; Aguilera, I., et al (2005). Am J Kidney Dis, 46, 345-350; Wichmann, I., et al (2006). Transfusion, 46, 1505-1509). In the case of some of these patients, it was confirmed that there was a correlation between the appearance of antibodies against GSTT1 and the development of an alloimmune response with adverse clinical symptoms (Aguilera, I., et al (2007). Tissue Antigens, 69, 396).

The methods for detecting anti-GSTT1 antibodies currently existing consist in indirect immunofluorescence and Western Blot testing (Rodriguez-Diaz, Y., et al (2006). Transplantation Proceedings, 38, 1467-1470; Aguilera, I., et al (2005). Transplantation Proceedings, 37, 3968-3969). In indirect immunofluorescence testing, different dilutions of the samples to be analysed are put in contact with cells or tissues carrying the specific antigen and secured on a slide. Thus, the antibodies present in the samples bind with the antigens exposed on the slide, to form antigen-antibody complexes. These complexes are detected by means of the binding with the same of other human anti-immunoglobulin antibodies marked with fluorescein, and observation under the microscope of the resulting fluorescence. The greatest dilution of sample capable of producing a fluorescence visible under the microscope to the human eye is established as the antibody count (Wheatley, S.P., et al (1998). Methods Cell Biol, 57, 313-332). In the Western Blot tests, the samples to be analysed are put in contact with nitrocellulose or polyvinylidene difluoride membranes, which contain cellular extracts from bacteria genetically modified with a cloning vector with the cDNA of the GSTT1, which allows it to express sufficient levels of the specific antigen. In this case, the antigen-antibody complexes formed are detected by means of the union with the same of other antibodies fused to an enzyme, alkaline phosphatase or peroxidise, which produces a colorimetric or chemiluminescent reaction in the presence of specific substrata. The results are interpreted as positive or negative for an antibody, depending on whether there is a reaction or not in the membrane (Simons, B., et al (2006). J. Immuno. L Methods, 315, 88-98). Both techniques, indirect immunofluorescence and Western Blot, are valid for detecting antibodies. However, they are semi-quantitative, complex, not easily automatizable and laborious analysis methods.

### EXPLANATION OF THE INVENTION

The first object of the present invention is an immunological method of analysis for the detection in biological fluids of antibodies directed against human GSTT1 (anti-GSTT1) which includes the following stages:
(a) Expression in a heterologous organism and purification of the hGSTT1 recombinant protein, a marker for antigen properties;
(b) Immobilisation of said hGSTT1 recombinant protein, a marker of antigen properties in a vehicle;
(c) Contact of said hGSTT1 recombinant protein, a marker for antigen properties with the biological fluid to be tested, in such conditions that the antibodies present in the biological fluid with specificity for said hGSTT1 recombinant protein bind with this material to form antigen-antibody complexes;
(d) Separation of unbound antibodies and other components of the biological fluid from the antigen-antibody complexes;
(e) Measurement of the amount of antigen-antibody complexes detected in the biological fluid, which will be positively correlated with the quantity of anti-GSTT1 antibodies defined by the positive control.

The hGSTT1 recombinant marker protein binds with a polypeptide sequence with at least 5 histidine amino acids, enabling its purification by Immobilized Metal Affinity Chromatography (IMAC), expressing itself in a microorganism, specifically, in an *E.coli* strain, carrier of the pcASB-GSTT1 plasmid.

The vehicle in which said hGSTT1 recombinant marker protein is immobilised is any surface allowing said immobilisation and the subsequent analysis of the binding of the anti-GSTT1 antibody, preferably wells of a multi-well culture plate, a nylon membrane, cellulose filter, nitrocellulose membrane, a coloured microparticle, a fluorescent microparticle, a glass surface or a metallic vehicle. The vehicle for the immobilisation of the hGSTT1 recombinant marker protein and the anti-GSTT1 antibody is a polypeptide micromatrix on a flat surface.

The detection stage of antigen-antibody complexes can be carried out using Western Blot, with the ELISA technique or by means of hGSTT1 recombinant marker protein-coated codified microparticles.

Likewise an object of the present invention is the use of the method of immunological analysis for detection in biological fluids of antibodies directed against GSTT1, for the diagnosis of pathological conditions associated with the presence in biological fluids of anti-GSTT1 antibodies, for the prognosis of onset of pathological conditions associated with the presence in biological fluids of antiGSTT1 antibodies, or for the follow-up and monitoring of conditions associated with the presence in biological fluids of anti-GSTT1.

The biological fluids in which the presence of anti-GSTT1 is detected are haemoderivatives and the pathological condition is the rejection of cells, tissues or organs from a transplant, grafting or transfusion, being especially indicated when the transplanted organ is the liver, the kidney, the heart, bone medulla or any other organ where the hGSTT1 sequence is expressed, and very specially indicated when the transplanted organ is the liver or the kidneys and likewise in patients receiving a blood transfusion or any haemoderivatives from donors expressing the hGSTT1 protein.

Last of all, also an object of the present invention is a tool kit for implementing the immunological analysis method for detection in biological fluids of antibodies directed against hGSTT1. The kit includes:
a) A vehicle in which the hGSTT1 recombinant antigen properties-marker protein has been immobilised,
b) A secondary human anti IgG antibody conjugated to indicator molecules, preferably enzymes, fluorophores, coloured microparticles or fluorescent microparticles that allow the binding of the anti-hGSTT1 antibody in a serum sample when placed in contact with the vehicle in which the hGSTT1 recombinant antigen properties-marker protein has been immobilised.
c) Means for detecting activity in the aforementioned indicator molecules conjugated to the secondary human IgG antibody.
d) A human control serum containing anti-hGSTT1 antibodies
e) A human control serum that does not contain human anti-hGSTT1 antibodies.

Preferably, the kit comprises:
a) A multi-well plate suitable for detecting antigen-antibody complexes using the ELISA technique, with the hGSTT1 recombinant antigen properties-marker protein immobilised in each of the wells.
b) A phial of secondary human anti IgG antibody conjugated to peroxydase.
c) A phial with a peroxydase enzymatic activity indicator substrate, preferably 3,3',5,5'-Tetramethylbenzidine.
d) A positive control consisting of a diluted sample of human serum with anti-hGSTT1 antibodies.
e) A negative control consisting in a diluted sample of human serum without anti-hGSTT1 antibodies.
f) Buffered solutions for making dilutions of the sample to be analysed, of the conjugated secondary antibody, of the controls and of the corresponding enzymatic indicator substrate.

Alternatively, the kit comprises:
a) A multi-well plate suitable for detecting antigen-antibody complexes using the ELISA technique, with the hGSTT1 recombinant antigen properties-marker protein immobilised in each of the wells.
b) A phial of secondary human anti IgG antibody conjugated to alkaline phosphatase.
c) A phial with a peroxydase enzymatic activity indicator substrate, preferably, p-nitrophenyl phosphate disodium.
d) A positive control consisting of a diluted sample of human serum with anti-hGSTT1 antibodies.
e) A negative control consisting in a diluted sample of human serum without anti-hGSTT1 antibodies.
f) Buffered solutions for making dilutions of the sample to be analysed, of the conjugated secondary antibody, of the controls and of the corresponding enzymatic indicator substrate.

### DESCRIPTION OF THE FIGURES

**Figure 1****.** Analysis of the purification of the human GSTT1 recombinant protein. M, molecular weight marker (BioRad); 1, insoluble proteins of the culture medium; 2, soluble proteins of the culture medium; 3, proteins not retained by the column; 4, 5, and 6, proteins expelled in the 1^{st} 2^{nd} and 3^{rd} wash, respectively; 7-18, successive elutions in imidazole gradient.
**Figure 2****.** Detection of anti-GSTT1 antibodies by means of a Western Blot test. M, molecular weight marker (BioRad); 1-6, different quantities of human GSTT1 recombinant: 500 ng, 1000 ng, 200 ng, 40 ng, 8 ng, and 1.6 ng respectively.
**Figure 3**. Detection of anti-GSTT1 antibodies by means of an indirect ELISA-type test. 1 and 2, samples carrying the anti-GSTT1 antibodies (GSTT1); 3 and 4, samples without GSTT1 antibodies (controls); 5, samples carrying antibodies against nuclear antigens (ANA); 6, sample carrying anti-HLA Class I antibodies (HLA-I); 7, sample carrying anti-HLA Class II antibodies (HLA-II); 8, sample carrying anti-HLA Class I and II antibodies (HLA-I/HLA-II). The data shown correspond to the mean of three values per sample analysed.
**Figure 4****.** Detection of markers for rejection in patients receiving a liver transplant. 1-8, samples analysed, as described in **Table 1;** 9, + Control, 10, - Control. The data shown correspond to the mean of three values per sample analysed.
**Figure 5****.** Detection of markers for rejection in patients receiving a kidney transplant. 1-8, samples analysed, as described in **Table 2;** 9, + Control, 10, - Control. The data shown correspond to the mean of three values per sample analysed.

### DETAILED DESCRIPTION OF THE INVENTION

The present invention refers to the use of the human GSTT1 expressed and isolated from a microorganism, preferably the *Escherichia coli* bacteria, for the diagnosis of the rejection of groups of heterologous cells transplanted from donors expressing said sequence to receivers who do not have said sequence. The group of cells may be tissue or organs, preferably relating to the liver or kidneys, or be part of biological fluids like the blood. The rejection would be detected by the presence of antibodies that bind with the hGSTT1 polypeptide sequence in the transplanted individual's serums. The diagnosis mechanism to determine the presence of antibodies against hGSTT1 would be the immobilisation of said polypeptide sequence, isolated to a surface, which can be the wells of a multi-well plate, a membrane, a fluorescent microparticle, coloured and/or magnetic, or any other type of habitual immobilisations carried out in immunological tests which enable the detection of the binding of transplanted individual's serum antibodies. Preferably, the test shall be made using an ELISA kit which comprises multi-well plates with the GSTT1 protein adhered to its wells, in which the sample of diluted human serum is placed and which would turn out to be a human anti-IgG antibody conjugated to an enzyme and a colorimetric or fluorometric substrate.

The object of the invention includes the use of a recombinant polypeptide material for the detection of markers for rejection of transplants, the antibodies directed against the human GSTT1 or anti-GSTT1 and their application in the diagnosis and monitoring of said rejection reactions. The method which is the object of the present invention improves the existing methods described in some scientific publications (Rodriguez-Diaz, Y., et al (2006). Transplantation Proceedings, 38, 1467-1470), as the use of human GSTT1 recombinant is defined as an immobilised antigen in a surface, and broadens the range of possibilities for its use in immunological tests and commercially available system.

To be precise, the present invention describes the use of a recombinant polypeptide material, the human GSTT1 protein, with a histidine tag (histag), which is super-produced in a micro-organism, preferably the *Escherichia coli* bacteria, modified with respect to that used in the previous state of the art and purified by means of immobilised metal affinity chromatography (IMAC) techniques.

The most novel characteristics of the object of the present invention can be summarised in the following points:
1. The detection of anti-GSTT1 antibodies as markers for the rejection of transplants can be done in biological samples from patients, preferably in the shape of blood serum.
2. The use of an isolated human GSTT1 recombinant (high degree of purity) as an antigen for the detection of human anti-GSTT1 antibodies minimises the risk of unspecific reactions with other antibodies directed against other proteins of bacterial origin, thus avoiding the appearance of false positives and errors in the interpretation of the samples analysed.
3. With an aim to facilitating the development of a sensitive, short-term, easily reproducible and automatizable mechanism for the detection of anti-GSTT1 antibodies, which even allows for the simultaneous analysis of a multitude of samples, the present invention includes the use of the human GSTT1 recombinant isolated in different habitual immunological tests for any technique in the field. Depending on the type of analysis:
   i) The isolated human GSTT1 (rhGSTT1) recombinant would be immobilised on a surface, which could be any of the following:
      (a) For ELISA tests, it would be immobilised on multi-well plates made from different polymers, already available on the market from a variety of manufacturers.
      (b) For Western Blot, Dot Blot tests or Immunochromatographic strip tests, it would be immobilised on papers or membranes made from cellulose derivatives such as nitrocellulose, or other polymeric compounds such as *polyvinylidene* difluoride or nylon.
      (c) For Luminex-type tests or other technologies based on multiple tests on fluid matrices, it would be immobilised on micro or nanoparticles of a variety of polymeric compounds such as polystyrene or silicone, or co-polymers.
      (d) For interaction tests in chromatographic vehicles or electrophoresis gels, it could be immobilised in polymers such as cellulose, polyacrylamide or agarose.
      (e) For biosensors, micromatrices on glass slides, and other kinds of advanced immunological tests, other surfaces that allow the immobilisation of proteins could be used.
   ii) The sample to be analysed is put into contact with said surface, so that the anti-GSTT1 antibodies of the sample would bind with the exposed human GSTT1 recombinant forming antigen-antibody complexes.
   iii) The antigen-antibody complexes would be detected using habitual techniques in immunological tests, such as:
      (a) Binding at human antibody constant region of other antibodies conjugated to fluorescent molecules or to enzymes generating fluorescent, luminescent or phosphorescent products, detectable under the microscope, scanner, flow cytometer or other devices habitually used in biotechnology.
      (b) The binding at human antibody constant region of other antibodies conjugated to enzymes, such as alkaline phosphatase or peroxydase, which produce a colorimetric, fluorescent or chemiluminescent reaction in the presence of specific substrata (Simons, B., et al (2006). J. Immunol Methods, 315, 88-89).
   iv) The detection of antigen-antibody complexes would be indicative of the presence of anti-GSTT1 antibodies in the sample analysed.

### Industrial application of the invention

Using the typical techniques of biotechnology, the present invention may be of use to:
1. Design kits or devices that allow the detection of anti-GSTT1 antibodies, based on the following techniques:
   a. ELISA (Enzyme-Linked ImmunoSorbent Assay)
   b. Western Blot or Dot Blot
   c. Immunochromatographic strips.
   d. Protein microarrays or arrays (micromatrices or matrices)
   e. Flow cytometer
   f. Paramagnetic microparticles for specific magnetic separation.
   g. Biosensors that detect the antigen-antibody binding in a quantitative fashion.
2. The use of these devices at the clinical level for the diagnosis and monitoring of alloimmune type reactions such as those caused by:
   a. Rejection of transplants of heterologous cells, tissues or organs in patients who do not express the human GSTT1.
   b. Haemolytic reactions (Transplacental passage) in foeti or the newly-born, not explained by known conventional reactions, Rh incompatibility or groups with fewer erythrocyturia.
   c. Cases of intolerance during pregnancy in allele negative GSTT1 mothers with allele positive GSTT1 foeti
   d. Intolerance to the administration of GSTT1 haemoderivatives in GSTT1 allele negative receivers.

### EMBODIMENT OF THE INVENTION

### Example 1. Production and purification of human GSTT1 recombinant

The present example describes how the human GSTT 1 recombinant protein can be obtained in an isolated fashion. A plasmid is constructed to express the human GSTT1 protein in bacteria bound to a 6-His tag. This can be used for the production and purification of the human GSTT1 recombinant by means of immobilised metals affinity chromatography techniques (IMAC).

The construction of the plasmid of expression in bacteria is done as follows. First of all, from a clone obtained by screening a Uni-Zap XR vector expression library (Aguilera, I., et al (2001). Clin Exp Immunol, 126, 535-539), the sequence codifying the GSTT1 enzyme is amplified using PCR (GenBank accession no. NM 000853), using Pfu DNA Polymerase (Bioneer) and, as primers, the oligonucleotides, CLONT1-F (SEQ ID no. 1) and Clont1-R (SEQ ID no. 2). The PCR product obtained is digested with the *Ban*HI and *Hind*III enzymes, and the resulting fragment is cloned in the pcASb plasmid (www.activemotif.com) previously digested with the same enzymes. Thus obtained is the pcASB-GSTT1 plasmid which expresses the human GSTT-1 recombinant bound to a His tag.

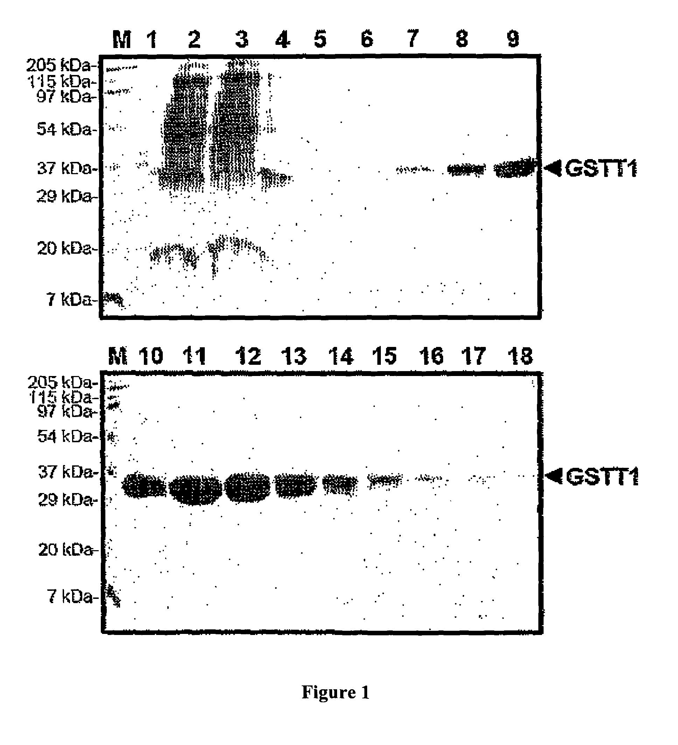

Subsequently, in order to purify the human GSTT1 recombinant, the pcASB-GSTT1 plasmid is transformed into the commercial bacterial strain REG-1 (Biomedal, ref. BS-3262). Using the transformant, cultures are made in LB liquid medium with 100 µg/ml ampicyline which are incubated at 37°C and shaken until an O.D at 600nm at 0.8-1.0 is reached, and are then incubated at 30°C and shaken for 4 hours more in the presence of 2 mM salicylate. The cells are gathered by centrifugation at 5000 g for 10 minutes at 4°C, and are resuspended in the solution of commercial lysis PROLYSE 1x (Biomedal, ref. RS-3406) supplemented with 0.25 mg/ml/lysozyme. The resulting suspension is incubated at ambient temperature for 15 minutes and shaken periodically; it is frozen at -80°C for 1 hour, and, following its defreezing to 37°C, it is sonicated by several sonication pulses in ice. After centrifugation at 9000g for 10 minutes at 4°C to eliminate cellular remains, the supernatant is removed with the culture's soluble proteins. This is passed twice through an IMAC resin column (HIS-Select™ Cartridge, Sigma-Aldrich) prebalanced with 6 column volumes of column balance buffer made up of 50 mM pH 8.0, 1.5 M NacCl, 0.1% Triton X-100, 20 mM imidazole potassium phosphate buffer. Then, the column is washed three times with 20 column volumes of column balance buffer. Finally, the human GSTT1 recombinant protein is eluded by repeatedly applying 1 column volume of buffers made up of 50 mM pH 8.0, 1.5 M NacCl, 0.1% Triton X-100, potassium phosphate buffer and which progressively increases the concentration of imidazole from 20 mM to 300 mM. The results of the purification are analysed by SDS-PAGE in gels with 12% of acrylamide stained by Coomassie (Figure 1), and the degree of purity of the elutions is determined by using the Experion™ System (Bio-Rad). Both the quantity produced (8.28 mg of purified protein per litre of culture) and the high degree of purity (above 96%) allow the application of the human GSTT1 recombinant in different types of tests, minimising the risk of non-specific reactions with other proteins which are bacterial in origin.

### Example 2. Method for the detection of anti-GSTT1 antibodies using the human GSTT1 recombinant purified in a Western Blot-type test as an antigen.

The following example illustrates how the presence of anti-GSTT1 antibodies can be detected. To do so, a Western Blot-type test using the human GSTT1 recombinant described in Example 1 as an antigen is carried out.

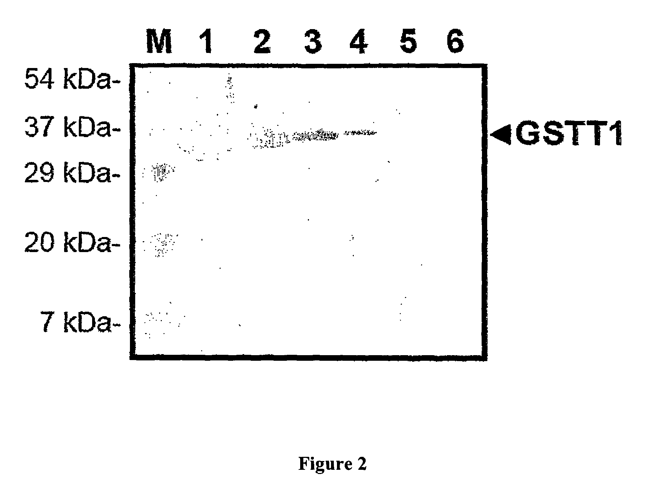

Different quantities of the human GSTT1 recombinant protein are subjected to a SDS-PAGE gel with 12% acrylamide. Then, the gel proteins are transferred to a polyvinylidene difluoride membrane (GE Healthcare) using a semi-dry transfer system (SV20-SDB, Sigma-Aldrich). After washing the membrane twice for 5 minutes in distilled water, it is dried at ambient temperature for 1 or 2 hours. Then the membrane is incubated at 4°C all night long with a sample of serum carrying anti-GSTT1 antibodies, diluted 1:100 in a buffer composed of 50 mM Tris-HCl ph 7.5, 150 mM NaCl, 0.02% Tween-20 and supplemented with 0.5% skimmed milk. After subjecting the membrane to three washes lasting 5 minutes in a wash buffer composed of 50 mM Tris-HCl pH 7.5, 150 mM NaCl, it is incubated at ambient temperature for 1 hour with a solution of antihuman IgG-AP conjugate antibody (Promega), diluted 1:1000 in a buffer composed of 50 mM Tris-HCl pH 7.5, 150 mM NaCl and supplemented with 0.5% skimmed milk. Finally, after subjecting the membrane to a further three washes lasting 5 minutes in a wash buffer, the antigen-antibody complexes formed on the membrane are revealed with a colorimetric substrate (SIGMA-FAST-BCIP/NBT system, Sigma-Aldrich). The results of this test are shown in Figure 2. As can be appreciated, the sample is capable of reacting and producing a positive signal from very small amounts (40ng) of human GSTT1 recombinant.

### Example 3. Development of a device for detecting anti-GSTT1 antibodies using the human GSTT1 recombinant as an antigen in an ELISA-type test.

The present example describes how to make a device, based on ELISA-type tests (Enzyme-Linked ImmunoSorbent Assay), which enables the detection of anti-GSTT1 antibodies by using the human GSTT1 recombinant described in **Example 1** immobilised on the surface of a multi-well plate.

In the first place, a solution with 1µg/ml of human GSTT1 recombinant dissolved in a buffer composed of 50 mM Tris-HCl pH 7.5, 150 mM NaCl is prepared. Then 100 µl of this GSTT1 solution and 100 µl of 100 mM pH 9.6 sodium carbonate buffer is added to each of the wells of a multi-well plate (Marxisorp ELISA plates, Nunc). Then the plate is incubated at 37°C for 1 hour, then at 4°C for one night and, finally, at 37°C for 30 minutes. After removing the contents, the wells are washed twice with 300 µl of a wash buffer made up of 50 mM Tris-HCl pH 7.5, 150 mM NaCl, 0.05% Tween-20, each wash is incubated at ambient temperature for 5 minutes and it must be verified that at the end no residual liquid remains in the wells. Then, 300 µl of a blocking solution made up of 5% skimmed milk dissolved in a wash buffer is added to each well, and the plate is incubated at ambient temperature for 1 or 2 hours. Meanwhile, dilutions 1:500 in blocking solution of samples of human serums carrying the following antibodies are prepared:
- Anti-GSTT1 antibodies (GSTT1)
- Antibodies against nuclear antigens (ANA)
- Anti-HLA Class I antibodies (HLA-I)
- Anti-HLA Class I antibodies (HLA-II)
- Anti-HLA Class I and Class II antibodies (HLA-I/HLA-II)
- Samples without antibodies (controls)

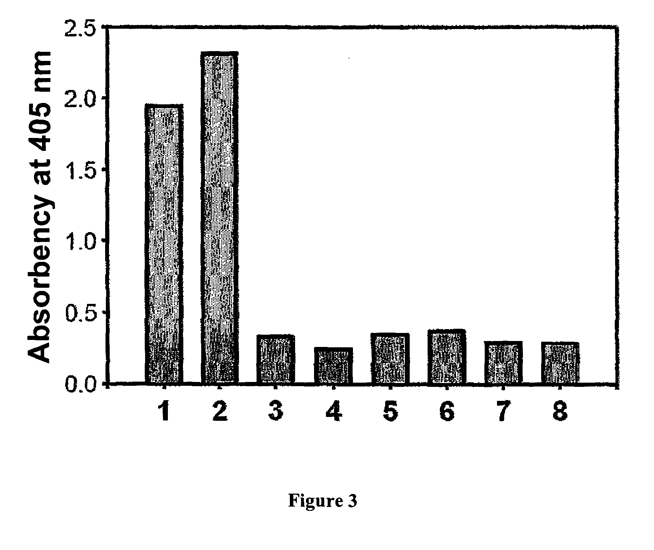

After removing the contents, 100 µl of the diluted samples are added to the wells and the plate is incubated at ambient temperature for 1 hour. After removing the contents, the wells are washed five times with 300 µl of wash buffer, making sure that no residual liquid remains in the wells. Then, 100 µl of a solution with anti-human IgG-AP antibody conjugate (Promega) is added to each well, diluted 1:5000 in a blocking solution, and the plate is incubated at ambient temperature for 1 hour. After removing the contents, the wells are washed five times with 300 µl of wash buffer, making sure that no residual liquid remains in the wells. Then, 100 µl of a solution with 1µlg/ml of p-nitrophenyl phosphate disodium (Aldrich) is added to each well, dissolved in a buffer composed of 100 mM Glycine, 1 mM MgCl₂, 1 mM ZnCl₂ pH 10.4, and the plate is incubated at ambient temperature for 1 hour in the dark. Having added 25 µl of 0.5 M NaOH to each well to stop the reaction, a reading is taken of absorbencies at a wavelength of 405 nm. The results of this test are illustrated in **Figure 3****.** As can be appreciated, the absorbency values obtained reveal significant differences (increments of up to five times) between the samples carrying anti-GSTT1 antibodies (GSTT1) and the samples lacking them (Controls). Moreover, the ELISA test does not present non-specific or cross-reactions with samples that contain other types of non-GSTT1 antibodies (ANA, HLA-I, HLA-II and HLA-I/HLA-II), thus revealing the high specificity of this method.

### Example 4. The diagnosis of reactions of rejection to liver transplant through detection of anti-GSTT1 antibodies by means of an ELISA-type test.

This examples illustrates how an ELISA-type device, which use the human GSTT1 recombinant, immobilised in multi-well plates, can allow the diagnosis and follow-up of patients with rejection to liver transplants by means of the detection of alloimmune reaction markers, anti-GSTT1 antibodies.

A battery of human serums, supplied by the Immunology Service of University Hospital Virgen del Rocio (Seville - Spain) and described in **Table 1,** from patients receiving livers with different counts of anti-GSTT1 antibodies, measured by indirect immunofluorescence techniques and/or Western Blot techniques, are used as samples in an ELISA-type test, as described in **Example 3.** Used as a reference are a sample carrying anti-GSTT1 antibodies (Control +) and another sample without antibodies (Control -).

**Table 1. Description of the samples analysed.**

| Sample no. | IIF | WB |
|---|---|---|
| 1 | >1/320 | + |
| 2 | >1/320 | + |
| 3 | >1/320 | + |
| 4 | 1/160 | + |
| 5 | 1/80 | + |
| 6 | 1/80 | + |
| 7 | 1/40 | - |
| 8 | - | - |

| | | |
|---|---|---|
| Abbreviations: IIF= anti-GSST1 count by indirect immunofluorescence; WB = presence of anti-GSTT1 in Wester Blot. | | |

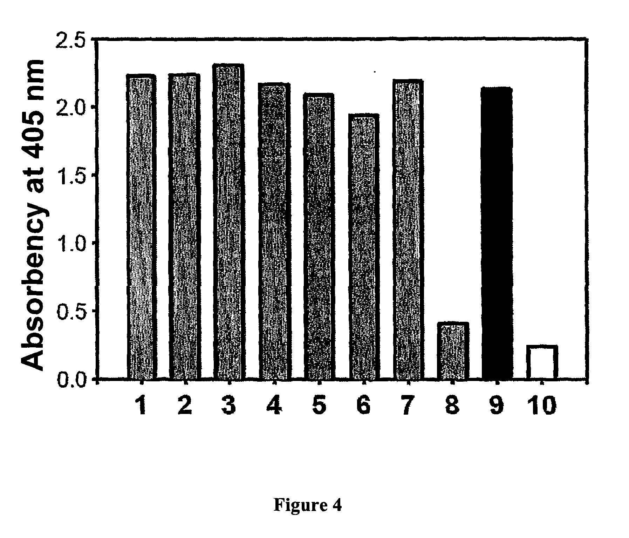

The results obtained are shown in Figure 4. As can be appreciated, the ELISA test proposed is capable of detecting the presence of anti-GSTT1 antibodies from very low counts (1/40). In addition, samples carrying anti-GSTT1 antibodies, which are considered negative in the Western Blot analysis (sample 7), present similar values to the positive control. This indicates the improvement in the sensitivity of the ELISA test with respect to other systems for the detection of anti-GSTT1 antibodies currently available, particularly because it also detects positivity in false negative serums in Western Blot as it only recognises native determinants.

### Example 5. Diagnosis of the reaction of rejection to a liver transplant through the detection of anti-GSTT1 antibodies by using an ELISA-type test.

The present example illustrates how an ELISA-type kit, which uses multi-well plates with the immobilised human GSTT1 recombinant, can allow the diagnosis and follow-up of patients with rejection to kidney transplants by means of the detection of alloimmune reaction markers, anti-GSTT1 antibodies.

Different human serums, supplied by the Immunology Service of University Hospital Virgen del Rocio (Seville - Spain) and described in **Table 2,** from patients receiving kidneys with different counts of anti-GSTT1 antibodies, measured by indirect immunofluorescence techniques and/or Western Blot techniques (Wichmann, I., et al (2006). Transfusion, 46, 1505-1509; Aguilera, I., et al (2005). Transplantation Proceedings, 37, 3968-3969), are used as in an ELISA-type test, as described in **Example 3**. Used as a reference are a sample carrying anti-GSTT1 antibodies (Control +) and another sample without antibodies (Control -).

**Table 2. Description of the samples analysed.**

| Sample no. | IIF | WB |
|---|---|---|
| 1 | 1/60 | + |
| 2 | ND | + |
| 3 | ND | + |
| 4 | 1/80 | - |
| 5 | 1/80 | - |
| 6 | ND | - |
| 7 | - | - |
| 8 | - | - |

| | | |
|---|---|---|
| Abbreviations: IIF= anti-GSST1 count by indirect immunofluorescence; WB = presence of anti-GSTT1 in Western Blot. ND: Tests done but not counted. | | |

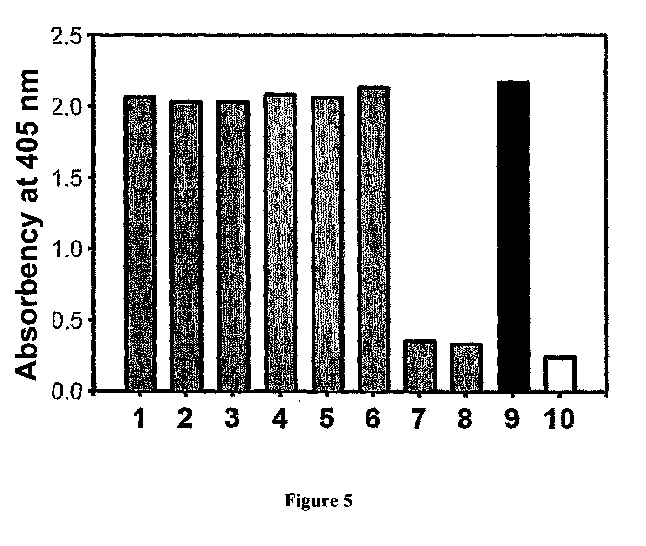

The results obtained are shown in **Figure 5****.** As can be appreciated, the ELISA test is capable of detecting the presence of anti-GSTT1 antibodies from very low counts (1/80) and which are considered negative in the Western Blot analysis. This indicates the improvement in the sensitivity of the ELISA test with respect to other systems for the detection of anti-GSTT1 antibodies currently available. As can be observed, the ELISA test described can detect the presence of anti-GSTT1 antibodies from very low counts (1/80). Moreover, samples carrying anti-GSTT1 antibodies which are considered negative in the Western Blot analysis (samples 4, 5 and 6) give similar values to the positive control. This indicates the sensibility of the ELISA test with respect to other systems for the detection of anti-GSTT1 antibodies currently available.

LIST OF SEQUENCES

SEQ ID Nº 1

LENGHT: 26 nucleotides.

TYPE: DNA.

SEQUENCE:

atggatccat gggtctggag ctctac 26

SEQ ID Nº 2

LENGHT: 26 nucleotides.

TYPE: DNA.

SEQUENCE:

gtaagctttc accggatcat ggccag 26

## Claims

1. Method of immunological analysis for the detection in biological fluids of antibodies directed against human GSTT1 (anti-hGSTT1) which includes the following stages:
a) expression in a heterologous organism and purification of the hGSTT1 recombinant protein, a marker for antigen properties;
b) immobilisation of said hGSTT1 recombinant protein and marker for antigen properties in a vehicle;
c) contact of said hGSTT1 recombinant protein, a marker for antigen properties, with the biological fluid to be tested, in such conditions that the antibodies present in the biological fluid with specificity for said hGSTT1 recombinant protein bind with this material to form antigen-antibody complexes;
d) separation of unbound antibodies and other components of the biological fluid from the antigen-antibody complexes;
e) measurement of the amount of antigen-antibody complexes detected in the biological fluid, which will be positively correlated with the quantity of anti-GSTT1 antibodies defined by the positive control.

2. A method of immunological analysis for the detection in biological fluids of antibodies directed against human GSTT1 according to Claim 1 **characterised in that** the hGSTT1 recombinant marker protein is expressed bound to a polypeptide sequence with at least 5 histidine amino acids enabling its purification by ionic interchange affinity chromatography.

3. A method of immunological analysis for the detection in biological fluids of antibodies directed against human GSTT1 according to claims 1 and 2, **characterised in that** the hGSTT1 recombinant marker protein is expressed and purified in a microorganism, in particular, in a *E.coli* strain, carrier of the pCASB-GSTT1 plasmid.

4. A method of immunological analysis for the detection in biological fluids of antibodies directed against human GSTT1 according to claims 1 to 3, **characterised in that** the vehicle in which said hGSTT1 recombinant marker protein is immobilised is any surface allowing said immobilisation and the subsequent analysis of the binding of the anti-GSTT1 antibody, preferably wells of a multi-well culture plate, a nylon membrane, cellulose filter, nitrocellulose membrane, a coloured microparticle, a fluorescent microparticle, a glass surface or a metallic vehicle.

5. A method of immunological analysis for the detection in biological fluids of antibodies directed against human GSTT1 according to claim 4, **characterised in that** the vehicle for the immobilisation of the hGSTT1 recombinant marker protein and the anti-hGSTT1 antibody is a polypeptide micromatrix on a flat surface.

6. A method of immunological analysis for the detection in biological fluids of antibodies directed against human GSTT1 according to claims 1 to 5, **characterised in that** the detection stage of antigen-antibody complexes is carried out using Western Blot.

7. A method of immunological analysis for the detection in biological fluids of antibodies directed against human GSTT1 according to claims 1 to 5, **characterised in that** the detection stage of antigen-antibody complexes is carried out using the ELISA technique.

8. A method of immunological analysis for the detection in biological fluids of antibodies directed against human GSTT1 according to claims 1 to 5,
**characterised in that** the detection stage of antigen-antibody complexes is carried out using hGSTT1 recombinant marker protein-coated codified microparticles.

9. Use of the method of immunological analysis for the detection in biological fluids of antibodies directed against GSTT1, for the diagnosis of pathological conditions associated with the presence in biological fluids of anti-GSTT1 antibodies, for the prognosis of onset of pathological conditions associated with the presence in biological fluids of anti-GSTT1 antibodies, or for the follow-up and monitoring of conditions associated with the presence in biological fluids of anti-GSTT1.

10. Use of the method of immunological analysis according to claim 9, **characterised in that** the biological fluids in which the presence of anti-GSTT1 is detected are haemoderivatives.

11. Use of the method of immunological analysis according to claims 9 and 10, **characterised in that** the pathological condition is the rejection of cells, tissues or organs from a transplant, grafting or transfusion.

12. Use of the method of immunological analysis according to claim 11 **characterised in that** the transplanted organ is the liver, the kidney, the heart, bone medulla or any other organ where the hGSTT1 sequence is expressed.

13. Use of the method of immunological analysis according to claim 12, **characterised in that** the transplanted organ is the liver.

14. Use of the method of immunological analysis according to claim 12, **characterised in that** the transplanted organ is the kidney.

15. Use of the method of immunological analysis according to claim 11, **characterised in that** the pathological condition is the rejection in patients receiving a blood transfusion or any haemoderivatives from donors expressing the hGSTT1 sequence.

16. Tool kit for implementing the immunological analysis method for detection in biological fluids of antibodies directed against GSTT1, **characterised in that** it includes:
a) a vehicle in which the hGSTT1 recombinant antigen properties-marker protein has been immobilised,
b) a secondary human anti IgG antibody conjugated to indicator molecules, preferably enzymes, fluorophores, coloured microparticles or fluorescent microparticles that allow the binding of the anti-hGSTT1 antibody in a serum sample when placed in contact with the vehicle in which the hGSTT1 recombinant antigen properties-marker protein has been immobilised.
c) means for detecting activity in the aforementioned indicator molecules conjugated to the secondary human IgG antibody
d) a human control serum containing anti-hGSTT1 antibodies
e) a human control serum that does not contain human anti-hGSTT1 antibodies.

17. A tool kit according to claim 16, **characterised in that** it is made up of:
a) a multi-well plate suitable for detecting antigen-antibody complexes using the ELISA technique, with the hGSTT1 recombinant antigen properties-marker protein immobilised in each of the wells;
b) a phial of secondary human anti IgG antibody conjugated to peroxydase
c) a phial with a peroxydase enzymatic activity indicator substrate, preferably 3,3',5,5'-Tetramethylbenzidine;
d) a positive control consisting of a diluted sample of human serum inactivated with anti-hGSTT1 antibodies;
e) a negative control consisting of a diluted sample of human serum without anti-hGSTT1 antibodies;
f) buffered solutions for making dilutions of the sample to be analysed, of the conjugated secondary antibody, of the controls and of the corresponding enzymatic indicator substrate.

18. A tool kit according to claim 16, **characterised in that** it is made up of:
a) a multi-well plate suitable for detecting antigen-antibody complexes using the ELISA technique, with the hGSTT1 recombinant antigen properties-marker protein immobilised in each of the wells;
b) a phial of secondary human anti IgG antibody conjugated to alkaline phosphatase;
c) a phial with a peroxydase enzymatic activity indicator substrate, preferably, p-nitrophenyl phosphate disodium;
d) a positive control consisting of a diluted sample of human serum inactivated with anti-GSTT1 antibodies;
e) a negative control consisting in a diluted sample of human serum without anti-hGSTT1 antibodies;
f) buffered solutions for making dilutions of the sample to be analysed, of the conjugated secondary antibody, of the controls and of the corresponding enzymatic indicator substrate.
